# FASCICULE DE BREVET EUROPEEN

(11) **EP 0 723 774 B1**
(45) Date de publication et mention de la délivrance du brevet: **06.12.2000**
(21) Numéro de dépôt: 96400007.9
(22) Date de dépôt: 02.01.1996
(51) Int. Cl.: A61K 7/48, A61K 38/22

(54) **Utilisation d'un antagoniste de CGRP dans une composition cosmétique, pharmaceutique ou dermatologique et composition obtenue**
Kosmetische, dermatologische oder pharmazeutische Zusammensetzung, enthaltend einen Antagonisten der CGRP
Cosmetic, dermatological or pharmaceutical composition comprising an antagonist of CGRP

(30) Priorité: 26.01.1995 FR 9500900
(43) Date de publication de la demande: 31.07.1996
(73) Titulaire: L'OREAL, 75008 Paris (FR)
(72) Inventeur: Breton, Lionel, F-78000 Versailles (FR); de Lacharriere, Olivier, F-75015 Paris (FR)
(74) Mandataire: Lhoste, Catherine

(56) Documents cités:
- WO-A-93/21911
- NEUROSCIENCE, vol. 48, no. 4, 1992, GB, pages 963-968, XP002002770 T. L. BUCKLEY ET AL: "The Partial Inhibition of Inflammatory Responses Induced By Capsaicin using the Fab Fragment of a Selective Calcitonin Gene-Related Peptide Antiserum in Rabbit Skin"
- BRITISH JOURNAL OF PHARMACOLOGY, vol. 110, no. 2, Octobre 1993, GB, pages 772-776, XP002002771 K. J. ESCOTT ET AL: "Effect of a calcitonin gene-related peptide antagonist (CGRP 8-37) on skin vasodilatation and oedema induced by stimulation of the rat saphenous nerve"
- BRITISH JOURNAL OF PHARMACOLOGY, vol. 104, no. 3, Novembre 1991, GB, pages 738-742, XP002002772 S. R. HUGHES ET AL: "A calcitonin gene-related peptide (CGRP) antagonist (CGRP 8-37) inhibits microvascular responses induced by CGRP and capsaicin in skin"

## Description

La présente invention se rapporte à l'utilisation d'un antagoniste de CGRP (peptide dérivé du gène de la calcitonine : Calcitonin Gene Related Peptide en langue anglosaxonne) dans une composition cosmétique, pharmaceutique ou dermatologique à application topique, destinée notamment au traitement des peaux sensibles, ainsi que la composition cosmétique contenant un antagoniste de CGRP en vue de diminuer voire éliminer les effets irritants de certains produits et notamment de certains actifs utilisés dans le domaine cosmétique, pharmaceutique ou dermatologique.

Il est connu que certaines peaux sont plus sensibles que d'autres. Les symptômes des peaux sensibles étaient jusqu'à présent mal caractérisés et le problème de ces peaux était, de ce fait, mal défini ; personne ne connaissait exactement le processus mis en cause dans la sensibilité -hyperréactivité cutanée non allergique- de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques.

Certains tests ont été essayés pour tenter de cerner les peaux sensibles, par exemple des tests à l'acide lactique et au DMSO qui sont connus pour être des substances irritantes : voir par exemple l'article de K. Lammintausta et al., Dermatoses, 1988, 36, pages 45-49; et l'article de T. Agner et J. Serup, Clinical and Experimental Dermatology, 1989, 14, pages 214-217. Mais ces tests ne permettaient pas de caractériser les peaux sensibles.

Par ailleurs, on assimilait les peaux sensibles à des peaux allergiques.

Du fait que l'on connaissait mal les caractéristiques des peaux sensibles, il était jusqu'à présent très difficile de les traiter, et on les traitait indirectement, par exemple en limitant dans les compositions cosmétiques l'emploi de produits à caractère irritant tels que les tensioactifs, les conservateurs ou les parfums ainsi que certains actifs.

La demanderesse a réalisé de nombreux tests cliniques et a su déterminer les symptômes liés aux peaux sensibles. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

La demanderesse a pu montrer en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique, il est aspécifique.

La demanderesse a maintenant trouvé que les peaux sensibles pouvaient être scindées en deux grandes formes cliniques, les peaux irritables et/ou réactives, et les peaux intolérantes.

Une peau irritable et/ou réactive est une peau qui réagit par un prurit, c'est-à-dire par des démangeaisons ou par des picotements, à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche avec ou sans dartres, ou à une peau qui présente un érythème.

Une peau intolérante est une peau qui réagit par des sensations d'échauffement, de tiraillements, de fourmillements et/ou de rougeurs, à différents facteurs tels que l'environnement, les émotions, les aliments. En général, ces signes sont associés à une peau hyperséborrhéique ou acnéique avec ou sans dartres, et à un érythème.

Les cuirs chevelus "sensibles" ont une sémiologie clinique plus univoque : les sensations de prurit et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à l'eau dure à forte concentration en calcaire et/ou aux variations de température.

Pour déterminer si une peau est sensible ou non, la demanderesse a également mis au point un test. En effet, après avoir effectué un grand nombre de tests dans le but de définir une peau sensible, elle a trouvé de manière surprenante qu'il existait un lien entre les personnes à peau sensible et celles qui réagissaient à une application topique de capsaïcine.

Le test à la capsaïcine consiste à appliquer sur environ 4 cm² de peau 0,05 ml d'une crème contenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

Jusqu'à présent, la capsaïcine était utilisée comme médicament, en particulier pour traiter les douleurs du zona. La capsaïcine provoque un relargage des neuropeptides à partir des fibres nerveuses sensitives, et en particulier du CGRP qui provient de terminaisons nerveuses de l'épiderme et du derme. La demanderesse a constaté que le schéma physiopathologique commun à tous les états des peaux sensibles était lié à une grande aptitude à libérer des neuropeptides et plus particulièrement du CGRP dans la peau. Les manifestations dysesthésiques qui sont provoquées par leur libération sont dites le neurogènes".

Ce CGRP est un élément chimique polypeptidique élaboré et libéré par une terminaison nerveuse. Le CGRP intervient notamment dans des maladies respiratoires et inflammatoires, dans des maladies allergiques et dans certaines maladies dermatologiques telles que l'eczéma ou le prurigo.

La demanderesse a maintenant découvert que l'une des caractéristiques essentielles des peaux sensibles était liée à la libération de CGRP et donc que l'utilisation d'antagonistes de CGRP pouvait permettre d'obtenir un effet préventif et/ou curatif des peaux sensibles.

Pour traiter les peaux sensibles, la demanderesse a donc envisagé d'utiliser des antagonistes de CGRP. Elle a en effet constaté de manière surprenante que l'incorporation d'un antagoniste de CGRP dans une composition cosmétique, pharmaceutique ou dermatologique permettait d'éviter l'irritation et/ou les sensations dysesthésiques et/ou les prurits de la peau et/ou des muqueuses et/ou l'érythème.

La présente invention a donc pour objet l'utilisation d'au moins un antagoniste de CGRP dans une composition contenant un milieu cosmétiquement ou pour la préparation d'une composition contenant un milieu dermatologiquement ou pharmaceutiquement acceptable, composition destinée au traitement des peaux sensibles des individus.

Certes, il est connu des documents Neuroscience, vol 48, N°4. pp. 963-968 (1992) de T. L. Buckley et al., Br. J. Pharmacol. (1991), vol. 104. pp. 738-742 de S. R. Hughes et al., et Br. J. Pharmacol. (1993). vol. 110, pp. 772-776 de K. J. Escott et al. que l'injection d'antagoniste de CGRP dans une veine ou la peau d'un animal inhibe les effets de la capsaïcine injectée ou appliquée topiquement à cet animal. Cependant, ces documents n'enseignent pas ni suggèrent que l'on puisse traiter les peaux sensibles par application topique d'antagoniste de CGRP.

Par ailleurs, il est connu du document WO-A-93/21911 d'utiliser des antagonistes de CGRP dans des compositions ophtalmiques pour empêcher la diminution de la pupille lors d'opération de l'oeil ou d'uvéite et non pour éliminer l'effet irritant de certains produits chimiques.

Plus précisément, l'invention permet de traiter les symptômes d'origine neurogène, dûs à un agent exogène susceptible de modifier les constantes biophysiques et biochimiques du tissu concerné (peau, muqueuses).

Par ailleurs, une peau sensible est une peau qui réagit plus facilement que d'autres à des facteurs extérieurs. Toute irritation d'une peau sensible commence par des signes subjectifs (picotement, échauffement,...) avant d'arriver à l'inflammation.

La présente invention a encore pour objet l'utilisation d'au moins un antagoniste de CGRP pour prévenir et/ou lutter contre les irritations cutanées et/ou les dartres et/ou les érythèmes et/ou les sensations d'échauffement et/ou de dysesthésie et/ou les prurits de la peau et/ou des muqueuses.

Un milieu cosmétiquement, dermatologiquement ou pharmaceutiquement acceptable est un milieu qui est compatible avec la peau, le cuir chevelu, les ongles et les muqueuses. La composition contenant l'antagoniste de CGRP peut être appliquée sur le visage, le cou, les cheveux et les ongles, ou toute autre zone cutanée du corps comme les grands plis (régions axillaires, sous-mammaires, plis du coude etc.).

La demanderesse définit un antagoniste de CGRP comme toute molécule d'origine organique ou minérale capable de produire une inhibition de la fixation réceptorielle du CGRP ou de produire une inhibition de la synthèse et/ou de la libération de CGRP par les fibres nerveuses sensitives.

Pour qu'une substance soit reconnue comme un antagoniste de CGRP, elle doit répondre notamment à la caractéristique suivante : avoir une activité pharmacologique antagoniste du CGRP, c'est-à-dire induire une réponse pharmacologique cohérente notamment dans l'un des tests suivants:
- la substance antagoniste doit diminuer la vasodilatation induite par la capsaïcine et/ou
- la substance antagoniste doit provoquer une inhibition de la libération de CGRP par les fibres nerveuses sensitives et/ou
- la substance antagoniste doit diminuer une inhibition de la contraction du muscle lisse du canal déférent induite par le CGRP.

En outre, l'antagoniste peut avoir une affinité pour les récepteurs au CGRP.

Personne jusqu'à ce jour n'avait établi un lien entre le CGRP et la peau sensible. Les signes cliniques de la peau sensible sont essentiellement subjectifs: picotements, fourmillements, prurits, tiraillements, échauffements, et érythèmes. Ces signes sont dûs à des facteurs extérieurs aspécifiques. Les symptômes apparaissent essentiellement localisés au visage, au cou et au cuir chevelu, mais peuvent apparaître aussi sur tout le corps.

On peut utiliser dans l'invention par exemple comme antagoniste de CGRP, le CGRP 8-37, un anticorps anti-CGRP.

Dans les compositions selon l'invention, l'antagoniste de CGRP est utilisé de préférence en une quantité allant de 0,000001 à 10 % en poids par rapport au poids total de la composition, et en particulier en une quantité allant de 0,0001 à 5 % en poids par rapport au poids total de la composition.

Les compositions selon l'invention peuvent se présenter sous toutes les formes galéniques normalement utilisées pour une application topique, notamment sous forme de solutions aqueuses, hydroalcooliques ou huileuses, de dispersions du type lotion ou sérum, de gels anhydres ou lipophiles, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps (par exemple crèmes de jour, crèmes de nuit, crèmes démaquillantes, crèmes de fond de teint, crèmes anti-solaires), des produits de maquillage comme les fonds de teint fluides, des laits de démaquillage, des laits corporels de protection ou de soin, des produits après-solaires sous forme de laits, des lotions, gels ou mousses pour le soin de la peau, comme des lotions de nettoyage ou de désinfection, des lotions anti-solaires, des lotions de bronzage artificiel, des compositions pour le bain, des compositions désodorisantes contenant un agent bactéricide, des produits après-rasage (gels ou lotions), des crèmes épilatoires, des compositions contre les piqûres d'insectes, des compositions anti-douleur, des compositions pour traiter l'acné, la peau hyperséborrhéique ou les dermites séborrhéiques ou des compositions pour traiter certaines symptomes de maladies de la peau comme les prurits sévères, la rosasée, l'acné, les ulcères de la jambe, le psoriasis, les pustules, les vergetures.

Les compositions selon l'invention peuvent également consister en des préparations solides constituant des savons ou des pains de nettoyage.

L'antagoniste de CGRP peut être aussi incorporé dans diverses compositions pour soins ou traitements capillaires, et notamment des shampooings éventuellement antiparasitaires, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, etc.

Les compositions de l'invention peuvent aussi être à usage bucco-dentaire, par exemple une pâte dentifrice ou un bain de bouche. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition de l'invention est une solution ou un gel huileux, la phase grasse peut représenter plus de 90 % du poids total de la composition.

De façon connue, la composition cosmétique de l'invention peut contenir également des adjuvants habituels dans le domaine cosmétique, pharmaceutique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine considéré, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, ou encore l'éthylcellulose, le polyéthylène

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon et des extraits végétaux, notamment ceux d'Aloe Vera.

Comme actifs lipophiles, on peut utiliser le rétinol (vitamine A) et ses dérivés, le tocophérol (vitamine E) et ses dérivés, les acides gras essentiels, les céramides, les huiles essentielles.

On peut entre autres associer les antagonistes de CGRP à des agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les estrogènes tels que l'estradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents antiviraux tels que l'acyclovir ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

De façon avantageuse, les antagonistes de CGRP sont associés à des produits et notamment des produits à effet secondaire irritant et notamment des actifs utilisés couramment dans le domaine cosmétique, pharmaceutique ou dermatologique. La présence d'un antagoniste de CGRP dans une composition cosmétique, pharmaceutique ou dermatologique contenant un actif ayant un effet irritant permet d'atténuer fortement, voire de supprimer cet effet irritant.

En particulier, les antagonistes de CGRP permettent notamment d'augmenter la quantité d'actif cosmétique, pharmaceutique ou dermatologique par rapport à la quantité normalement utilisée, en vue d'une efficacité améliorée.

Aussi, l'invention a encore pour objet une composition contenant un milieu cosmétiquement, pharmaceutiquement ou dermatologiquement acceptable et au moins un produit à effet secondaire irritant, caractérisée en ce qu'elle contient au moins un antagoniste de CGRP.

Les produits irritants auxquels s'applique l'invention sont notamment les parfums, les tensioactifs (ioniques ou non-ioniques), les conservateurs, certains filtres solaires, les solvants organiques, les solutions alcooliques et certains actifs cosmétiques, pharmaceutiques ou dermatologiques.

En particulier, les produits à effet secondaire irritant sont choisis parmi les α-hydroxy-acides(glycolique, lactique, malique, citrique, tartrique, mandélique ), les β-hydroxy-acides (acide salicylique et ses dérivés), les α-céto-acides, les β-cétoacides, les rétinoïdes (rétinol et ses esters, rétinal, acide rétinoïque et ses dérivés, rétinoïdes, notamment ceux décrits dans les documents FR-A-2 570 377, EP-A-199636, EP-A-325540, EP-A-402072), les anthralines (dioxyanthranol), les anthranoïdes, les peroxydes, le minoxidil, les sels de lithium, les antimétabolites, la vitamine D et ses dérivés, les teintures ou colorants capillaires (paraphénylènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les dépigmentants (hydroquinone), les actifs antipoux (pyréthrine).

L'emploi d'un antagoniste de CGRP permet notamment de multiplier de 2 à 10 fois la quantité de produit, et plus spécialement d'actif à effet secondaire irritant par rapport à l'état de la technique, sans ressentir tous les inconforts mentionnés ci-dessus. Ainsi, on peut utiliser les hydroxyacides jusqu'à 50 % du poids de la composition ou les rétinoïdes jusqu'à 5 %, sans aucune gène.

La présente invention a en outre pour objet un procédé de traitement non-thérapeutique, caractérisé par le fait que l'on applique sur la peau, sur le cuir chevelu, et/ou sur les muqueuses, une composition telle que décrite ci-dessus contenant au moins un antagoniste de CGRP dans un milieu cosmétiquement acceptable.

Le procédé de traitement non-thérapeutique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions après-solaires sur la peau ou sur les cheveux secs, application d'une lotion pour cheveux sur cheveux mouillés, de shampooings, ou encore application de dentifrice sur les gencives.

Les exemples suivants illustrent l'invention. Dans ces exemples, les proportions indiquées sont des pourcentages en poids.

| Exemple 1 : lotion démaquillante pour le visage | |
|---|---|
| CGRP 8-37 | 0,50 |
| Antioxydant | 0,05 |
| Isopropanol | 40,00 |
| Conservateur | 0,30 |
| Eau | qsp 100 % |

| Exemple 2: lotion démaquillante pour le visage | |
|---|---|
| CGRP 8-37 | 0,0001 |
| Antioxydant | 0,05 |
| Isopropanol | 40,00 |
| Conservateur | 0,30 |
| Eau | qsp 100 % |

| Exemple 3 : Gel pour le soin du visage | |
|---|---|
| Anticorps Anti-CGRP | 0,05 |
| Hydroxypropylcellulose (Klucel H vendu par la société | |
| Hercules) | 1,00 |
| Antioxydant | 0,05 |
| Isopropanol | 40,00 |
| Conservateur | 0,30 |
| Eau | qsp 100 % |

| Exemple 4 : Gel pour le traitement del'acné | |
|---|---|
| Anticorps Anti-CGRP | 0,10 |
| Acide all trans rétinoïque | 0,05 |
| Hydroxypropylcellulose (Klucel H vendu par la société Hercules) | 1,00 |
| Antioxydant | 0,05 |
| Isopropanol | 40,00 |
| Conservateur | 0,30 |
| Eau | qsp 100 % |

| Exemple 5 : Crème de soin du visage (émulsion huile-dans- eau) | |
|---|---|
| CGRP 8-37 | 0,02 |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |

| Exemple 6 : Shampooing | |
|---|---|
| Lauryl éther sulfate de Na (2,2 OE) | 12,00 |
| Anticorps Anti-CGRP | 0,02 |
| Hydroxypropylcellulose (Klucel H vendu par la société Hercules) | 1,00 |
| Parfum | 0,50 |
| Conservateur | 0,30 |
| Eau | qsp 100 % |

| Exemple 7 : Crème de soin antirides pour le visage (émulsion huile-dans-eau) | |
|---|---|
| CGRP 8-37 | 0,15 |
| Stéarate de glycérol | 2,00 |
| Polysorbate 60 (Tween 60 vendu par la société ICI) | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |

| Exemple 8 : Gel émulsionné de soin contre les piqûres d'insectes (émulsion huile-dans-eau) | |
|---|---|
| Cyclomethicone | 3,00 |
| Huile de Purcellin (vendue par la Société Dragocco) | 7,00 |
| PEG-6/PEG-32/Glycol Stéarate (Tefose^{R} 63 de Gattefosse) | 0,30 |
| Anticorps Anti CGRP | 0,02 |
| Conservateur | 0,30 |
| Parfum | 0,40 |
| Carbomer | 0,60 |
| Crotamiton | 5,00 |
| Acide glycyrrhétinique | 2,00 |
| Alcool éthylique | 5,00 |
| Triéthanolamine | 0,20 |
| Eau | qsp 100 % |

| Exemple 9 : Gel anti-douleur | |
|---|---|
| CGRP 8-37 | 0,03 |
| Hydroxypropylcellulose (Klucel H vendu par la société Hercules) | 1,00 |
| Antioxydant | 0,05 |
| Chlorhydrate de lidocaïne | 2,00 |
| Isopropanol | 40,00 |
| Conservateur | 0,30 |
| Eau | qsp 100 % |

| Exemple 10 : Crème de soin de la rosacée du visage (émulsion huile-dans-eau) | |
|---|---|
| CGRP 8-37 | 0,25 |
| Stéarate de glycérol | 2,00 |
| Polysorbate 60 (Tween 60 vendu par la société ICI) | 1,00 |
| Acide stéarique | 1,40 |
| Métronidazole | 1,00 |
| Triéthanolamine | 0,70 |
| Carbomer | 0,40 |
| Fraction liquide du beurre de karité | 12,00 |
| Huile de vaseline | 12,00 |
| Antioxydant | 0,05 |
| Parfum | 0,50 |
| Conservateur | 0,30 |
| Eau | qsp 100 % |

| Exemple 11 : Crème de soin de l'érythème solaire (émulsion huile-dans-eau) | |
|---|---|
| Anticorps Anti CGRP | 0,25 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,50 |
| Conservateur | 0,30 |
| Eau | qsp 100 % |

## Revendications

1. Utilisation non-thérapeutique d'au moins un antagoniste de CGRP dans une composition contenant un milieu cosmétiquement acceptable.

2. Utilisation d'au moins un antagoniste de CGRP pour la préparation d'une composition contenant un milieu dermatologiquement ou pharmaceutiquement acceptable destinée au traitement topique des peaux sensibles des individus.

3. Utilisation selon la revendication 1 ou 2, caractérisée en ce que l'antagoniste de CGRP est une molécule qui diminue la vasodilatation induite par la capsaïcine.

4. Utilisation selon l'une des revendications 1 à 3, caractérisée en ce que l'antagoniste de CGRP est une molécule qui diminue une inhibition de la contraction du muscle lisse du canal déférent induite par le CGRP.

5. Utilisation selon l'une quelconque des revendications précédentes, caractérisée en ce que l'antagoniste de CGRP est choisie parmi le CGRP 8-37 et les anticorps anti-CGRP.

6. Utilisation selon l'une quelconque des revendications précédentes, caractérisée en ce que l'antagoniste de CGRP est utilisé en une quantité allant de 0,000001 à 1 0 % en poids par rapport au poids total de la composition.

7. Utilisation selon l'une quelconque des revendications précédentes, caractérisée en ce que l'antagoniste de CGRP est utilisé en une quantité allant de 0,0001 à 5 % en poids par rapport au poids total de la composition.

8. Utilisation selon l'une quelconque des revendications précédentes, caractérisée en ce que le milieu cosmétiquement, pharmaceutiquement ou dermatologiquement acceptable est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules.

9. Utilisation selon l'une quelconque des revendications précédentes, caractérisée en ce que la composition contient au moins un agent choisi parmi les agents antibactériens, antiparasitaires, antifongiques, anti-inflammatoires, antiprurigineux, anesthésiques, antiviraux, kératolytiques, anti-radicaux libres, antiséborrhéiques, antipelliculaires, antiacnéiques et/ou les agents modulant la différenciation et/ou la prolifération et/ou la pigmentation cutanée.

10. Utilisation selon la revendication précédente, caractérisée en ce que l'agent est le chlorhydrate de lidocaïne, un antiparasitaire ou un anti-inflammatoire non stéroïdien.

11. Utilisation d'au moins un antagoniste de CGRP pour la préparation d'une composition contenant un milieu dermatologiquement ou pharmaceutiquement acceptable destinée au traitement topique des irritations cutanées et/ou des dartres et/ou des érythèmes et/ou des sensations dysesthésiques et/ou des sensations d'échauffement et/ou des prurits de la peau et/ou des muqueuses.

12. Utilisation selon la revendication 11, caractérisée en ce que l'antagoniste de CGRP est utilisé en une quantité allant de 0,000001 à 10 % en poids par rapport au poids total de la composition.

13. Utilisation selon l'une quelconque des revendications 11 et 12, caractérisée en ce que l'antagoniste de CGRP est utilisé en une quantité allant de 0,0001 à 5 % en poids par rapport au poids total de la composition.

14. Utilisation selon l'une quelconque des revendications 11 à 13, caractérisée en ce que l'antagoniste de CGRP est une molécule qui diminue la vasodilatation induite par la capsaïcine.

15. Utilisation selon l'une quelconque des revendications 10 à 14, caractérisée en ce que l'antagoniste de CGRP est une molécule qui diminue une inhibition de la contraction du muscle lisse du canal déférent induite par le CGRP.

16. Procédé de traitement cosmétique, caractérisé en ce que l'on applique sur la peau, sur le cuir chevelu et/ou sur les muqueuses une composition contenant au moins un antagoniste de CGRP dans un milieu cosmétiquement acceptable.

17. Procédé de traitement cosmétique pour traiter les peaux sensibles, caractérisé en ce que l'on applique sur la peau une composition contenant au moins un antagoniste de CGRP dans un milieu cosmétiquement acceptable.

18. Procédé selon la revendication 16 ou 17, caractérisée en ce que l'antagoniste de CGRP est une molécule qui diminue la vasodilatation induite par la capsaïcine.

19. Procédé selon l'une des revendications 16 à 18, caractérisé en ce que l'antagoniste de CGRP est une molécule qui diminue une inhibition de la contraction du muscle lisse du canal déférent induite par le CGRP.

20. Procédé selon l'une quelconque des revendications 16 à 19, caractérisé en ce que l'antagoniste de CGRP est choisi parmi le CGRP 8-37 et les anticorps anti-CGRP.

21. Procédé selon l'une quelconque des revendication 16 à 20, caractérisé en ce que l'antagoniste de CGRP est utilisé en une quantité allant de 0,000001 à 10 % en poids par rapport au poids total de la composition.

22. Procédé selon l'une quelconque des revendications 16 à 21, caractérisé en ce que l'antagoniste de CGRP est utilisé en une quantité allant de 0,00001 à 5 % en poids par rapport au poids total de la composition.

23. Composition topique contenant un milieu cosmétiquement, pharmaceutiquement ou dermatologiquement acceptable et au moins un produit à effet secondaire irritant, caractérisée en ce qu'elle contient, en outre, au moins un antagoniste de CGRP tel qu'il atténue l'effet irritant dudit produit.

24. Composition selon la revendication 24, caractérisée que l'antagoniste de CGRP est une molécule qui diminue la vasodilatation induite par la capsaïcine.

25. Composition selon la revendication 23 ou 24, caractérisée en ce que l'antagoniste de CGRP est une molécule qui diminue une inhibition de la contraction du muscle lisse du canal déférent induite par le CGRP.

26. Composition selon l'une quelconque des revendications 23 à 25, caractérisée en ce que l'antagoniste de CGRP est choisi parmi les molécules organiques ou minérales.

27. Composition selon l'une quelconque des revendications 23 à 26, caractérisée en ce que l'antagoniste de CGRP est utilisé en une quantité allant de 0,000001 à 10 % en poids par rapport au poids total de la composition.

28. Composition selon l'une quelconque des revendications 23 à 27, caractérisée en ce que l'antagoniste de CGRP est utilisé en une quantité allant de 0,0001 à 5 % en poids par rapport au poids total de la composition.

29. Composition selon l'une quelconque des revendications 23 à 28, caractérisée en ce que le produit à effet secondaire irritant est choisi parmi les α-hydroxyacides, les β-hydroxyacides, les α-céto-acides, les β-céto-acides, les rétinoïdes, les anthralines, les anthranoïdes, les peroxydes, le minoxidil, les sels de lithium, les antimétabolites, la vitamine D et ses dérivés, les dépigmentants, les solvants, les parfums, les conservateurs, les tensioactifs, les solutions alcooliques.

30. Composition selon l'une quelconque des revendications 23 à 29, caractérisée en ce qu'elle contient en outre au moins un agent choisi parmi les agents antibactériens, antiparasitaires, antifongiques, anti-inflammatoires, antiprurigineux, anesthésiques, antiviraux, kératolytiques, anti-radicaux libres, antiséborrhéiques, antipelliculaires, antiacnéiques et/ou les agents modulant la différenciation et/ou la prolifération et/ou la pigmentation cutanée.

31. Composition selon l'une quelconque des revendications 23 à 30, caractérisée en ce que la composition constitue une composition de nettoyage, de protection, de traitement, de bronzage artificiel ou de soin pour le visage, les mains, les pieds, les grands plis anatomiques ou le corps, un produit de maquillage, un lait de démaquillage, un produit après-solaire, une composition pour le bain, une composition désodorisante, pour traiter les rides, l'acné ou la peau hyperséborrhéique ou les dermites séborrhéique, un produit après-rasage, une crème épilatoire, une composition contre les piqûres d'insectes, une composition anti-douleur, pour le soin ou le traitement capillaire, de shampooing, de mise en plis, de produit coiffant, de teinture ou de permanente, une composition antichute, bucco-dentaire.

## Patentansprüche

1. Nicht therapeutische Verwendung mindestens eines CGRP-Antagonisten in einer Zusammensetzung, die ein kosmetisch akzeptables Medium enthält.

2. Verwendung mindestens eines CGRP-Antagonisten zur Herstellung einer Zusammensetzung, die ein dermatologisch oder pharmazeutisch akzeptables Medium enthält und zur topischen Behandlung von empfindlicher Haut von Personen vorgesehen ist.

3. Verwendung nach Anspruch 1 oder 2, dadurch gekennzeichnet, daß der CGRP-Antagonist ein Molekül ist, das die durch Capsaicin hervorgerufene Vasodilatation verringert.

4. Verwendung nach einem der Ansprüche 1 bis 3, dadurch gekennzeichnet, daß der CGRP-Antagonist ein Molekül ist, das eine Inhibierung der Kontraktion der glatten Muskulatur des Samenleiters, die durch CGRP hervorgerufen wird, vermindert.

5. Verwendung nach einem der vorhergehenden Ansprüche, dadurch gekennzeichnet, daß der CGRP-Antagonist unter CGRP 8-37 und anti-CGRP-Antikörpern ausgewählt ist.

6. Verwendung nach einem der vorhergehenden Ansprüche, dadurch gekennzeichnet, daß der CGRP-Antagonist in einem Mengenanteil von 0,000001 bis 10 Gew.-%, bezogen auf das Gesamtgewicht der Zusammensetzung, verwendet wird.

7. Verwendung nach einem der vorhergehenden Ansprüche, dadurch gekennzeichnet, daß der CGRP-Antagonist in einem Mengenanteil von 0,0001 bis 5 Gew.-%, bezogen auf das Gesamtgewicht der Zusammensetzung, verwendet wird.

8. Verwendung nach einem der vorhergehenden Ansprüche, dadurch gekennzeichnet, daß das kosmetisch, pharmazeutisch oder dermatologisch akzeptable Medium eine wäßrige, ölige oder wäßrig-alkoholische Lösung, eine Wasser-in-Öl-Emulsion, eine Öl-in-Wasser-Emulsion, eine Mikroemulsion, ein wäßriges Gel, ein wasserfreies Gel, ein Serum, eine Vesikeldispersion, eine Mikrokapseldispersion oder eine Mikropartikeldispersion ist.

9. Verwendung nach einem der vorhergehenden Ansprüche, dadurch gekennzeichnet, daß die Zusammensetzung mindestens einen Wirkstoff enthält, der unter den antibakteriellen Mitteln, antiparasitären Mitteln, Mitteln gegen Pilze, entzündungshemmenden Mitteln, Mitteln gegen Juckreiz, Anästhetika, Mitteln gegen Viren, Keratolytika, Mitteln gegen freie Radikale, Mitteln gegen Seborrhoe, Mitteln gegen Schuppen, Mitteln gegen Akne und/oder Mitteln, die die Differenzierung und/oder Proliferation und/oder Pigmentierung der Haut modulieren, ausgewählt ist.

10. Verwendung nach dem vorhergehenden Anspruch, dadurch gekennzeichnet, daß das Mittel das Lidocain-Hydrochlorid, ein antiparasitäres Mittel oder ein nicht steroidales entzündungshemmendes Mittel ist.

11. Verwendung mindestens eines CGRP-Antagonisten zur Herstellung einer Zusammensetzung, die ein dermatologisch oder pharmazeutisch akzeptables Medium enthält und zur topischen Behandlung von Hautreizungen und/oder Flechten und/oder Erythemen und/oder dysästhesischen Empfindungen und/oder Hitzeempfindungen und/oder Juckreiz der Haut und/oder der Schleimhäute vorgesehen ist.

12. Verwendung nach Anspruch 11, dadurch gekennzeichnet, daß der CGRP-Antagonist in einem Mengenanteil von 0,000001 bis 10 Gew.-%, bezogen auf das Gesamtgewicht der Zusammensetzung, verwendet wird.

13. Verwendung nach einem der Ansprüche 11 und 12, dadurch gekennzeichnet, daß der CGRP-Antagonist in einem Mengenanteil von 0,0001 bis 5 Gew.-%, bezogen auf das Gesamtgewicht der Zusammensetzung, verwendet wird.

14. Verwendung nach einem der Ansprüche 11 bis 13, dadurch gekennzeichnet, daß der CGRP-Antagonist ein Molekül ist, das die durch Capsaicin hervorgerufene Vasodilatation verringert.

15. Verwendung nach einem der Ansprüche 11 bis 14, dadurch gekennzeichnet, daß der CGRP-Antagonist ein Molekül ist, das eine Inhibierung der Kontraktion der glatten Muskulatur des Samenleiters, die durch CGRP hervorgerufen wird, vermindert.

16. Verfahren zur kosmetischen Behandlung, dadurch gekennzeichnet, daß auf die Haut, die Kopfhaut und/oder die Schleimhäute eine Zusammensetzung aufgetragen wird, die mindestens einen CGRP-Antragonisten in einem kosmetisch akzeptablen Medium enthält.

17. Verfahren zur kosmetischen Behandlung von empfindlicher Haut, dadurch gekennzeichnet, daß auf die Haut eine Zusammensetzung aufgetragen wird, die mindestens einen CGRP-Antragonisten in einem kosmetisch akzeptablen Medium enthält.

18. Verfahren nach Anspruch 16 oder 17, dadurch gekennzeichnet, daß der CGRP-Antagonist ein Molekül ist, das die durch Capsaicin hervorgerufene Vasodilatation verringert.

19. Verfahren nach einem der Ansprüche 16 bis 18, dadurch gekennzeichnet, daß der CGRP-Antagonist ein Molekül ist, das eine Inhibierung der Kontraktion der glatten Muskulatur des Samenleiters, die durch CGRP hervorgerufen wird, vermindert.

20. Verfahren nach einem der Ansprüche 16 bis 19, dadurch gekennzeichnet, daß der CGRP-Antagonist unter CGRP 8-37 und anti-CGRP-Antikörpern ausgewählt ist.

21. Verfahren nach einem der Ansprüche 16 bis 20, dadurch gekennzeichnet, daß der CGRP-Antagonist in einem Mengenanteil von 0,000001 bis 10 Gew.-%, bezogen auf das Gesamtgewicht der Zusammensetzung, verwendet wird.

22. Verfahren nach einem der Ansprüche 16 bis 21, dadurch gekennzeichnet, daß der CGRP-Antagonist in einem Mengenanteil von 0,00001 bis 5 Gew.-%, bezogen auf das Gesamtgewicht der Zusammensetzung, verwendet wird.

23. Topische Zusammensetzung, die in einem kosmetisch, pharmazeutisch oder dermatologisch akzeptablen Medium mindestens ein Produkt mit reizender Nebenwirkung enthält, dadurch gekennzeichnet, daß sie ferner mindestens einen CGRP-Antagonisten enthält, der die reizende Wirkung des Produktes abschwächt.

24. Zusammensetzung nach Anspruch 23, dadurch gekennzeichnet, daß der CGRP-Antagonist ein Molekül ist, das die durch Capsaicin hervorgerufene Vasodilatation verringert.

25. Zusammensetzung nach Anspruch 23 oder 24, dadurch gekennzeichnet, daß der CGRP-Antagonist ein Molekül ist, das eine Inhibierung der Kontraktion der glatten Muskulatur des Samenleiters, die durch CGRP hervorgerufen wird, vermindert.

26. Zusammensetzung nach einem der Ansprüche 23 bis 25, dadurch gekennzeichnet, daß der CGRP-Antagonist unter organischen oder anorganischen Molekülen ausgewählt ist.

27. Zusammensetzung nach einem der Ansprüche 23 bis 26, dadurch gekennzeichnet, daß der CGRP-Antagonist in einem Mengenanteil von 0,000001 bis 10 Gew.-%, bezogen auf das Gesamtgewicht der Zusammensetzung, verwendet wird.

28. Zusammensetzung nach einem der Ansprüche 23 bis 27, dadurch gekennzeichnet, daß der CGRP-Antagonist in einem Mengenanteil von 0,0001 bis 5 Gew.-%, bezogen auf das Gesamtgewicht der Zusammensetzung, verwendet wird.

29. Zusammensetzung nach einem der Ansprüche 23 bis 28, dadurch gekennzeichnet, daß das Produkt mit reizender Nebenwirkung unter den α-Hydroxysäuren, β-Hydroxysäuren, α-Ketosäuren, β-Ketosäuren, Retinoiden, Anthralinen, Anthranoiden, Peroxiden, Minoxidil, Lithiumsalzen, Antimetaboliten, Vitamin D und seinen Derivaten, Depigmentierungsmitteln, Lösungsmitteln, Parfums, Konservierungsmitteln, grenzflächenaktiven Stoffen und alkoholischen Lösungen ausgewählt ist.

30. Zusammensetzung nach einem der Ansprüche 23 bis 29, dadurch gekennzeichnet, daß sie ferner mindestens ein Mittel enthält, das unter den antibakteriellen Mitteln, antiparasitären Mitteln, Mitteln gegen Pilze, entzündungshemmenden Mitteln, Mitteln gegen Juckreiz, Anästhetika, Mitteln gegen Viren, Keratolytika, Mitteln gegen freie Radikale, Mitteln gegen Seborrhoe, Mitteln gegen Schuppen, Mitteln gegen Akne und/oder Mitteln, die die Differenzierung und/oder Proliferation und/oder Pigmentierung der Haut modulieren, ausgewählt ist.

31. Zusammensetzung nach einem der Ansprüche 23 bis 30, dadurch gekennzeichnet, daß es sich bei den Zusammensetzung um Zusammensetzungen zur Reinigung, zum Schutz, zur Behandlung, zur künstlichen Bräunung oder zur Pflege des Gesichts, der Hände, der Füße, der Körperfalten oder des Körpers, Produkte zum Schminken, Milche zum Abschminken, After-sun-Produkte, Zusammensetzungen für das Bad, desodorierende Zusammensetzungen, Zusammensetzungen zur Behandlung von Falten, Akne, Hyperseborrhoehaut oder seborrhoischer Dermatitis, Produkte zur Anwendung nach der Rasur, Haarentfernungscremes, Zusammensetzungen gegen Insektenstiche, schmerzstillende Zusammensetzungen, Zusammensetzungen zur Pflege oder Behandlung der Haare, Haarwaschmittel, Zusammensetzungen für Wasserwellen, Frisierprodukte, Zusammensetzungen zum Färben oder Entfärben, Zusammensetzungen gegen Haarausfall und Zusammensetzungen für den Mund-Zahn-Bereich handelt.

## Claims

1. Non-therapeutic use of at least one CGRP antagonist in a composition containing a cosmetically acceptable medium.

2. Use of at least one CGRP antagonist for preparing a composition containing a dermatologically or pharmaceutically acceptable medium and intended for the topical treatment of the sensitive skins of individuals.

3. Use according to Claim 1 or 2, characterized in that the CGRP antagonist is a molecule which reduces the vasodilation induced by capsaicin.

4. Use according to one of Claims 1 to 3, characterized in that the CGRP antagonist is a molecule which reduces an inhibition of the contraction of the smooth muscle of the deferent canal induced by CGRP.

5. Use according to any one of the preceding claims, characterized in that the CGRP antagonist is chosen from CGRP 8-37 and anti-CGRP antibodies.

6. Use according to any one of the preceding claims, characterized in that the CGRP antagonist is used in an amount ranging from 0.000001 to 10% by weight relative to the total weight of the composition.

7. Use according to any one of the preceding claims, characterized in that the CGRP antagonist is used in an amount ranging from 0.0001 to 5% by weight relative to the total weight of the composition.

8. Use according to any one of the preceding claims, characterized in that the cosmetically, pharmaceutically or dermatologically acceptable medium is an aqueous, oily or aqueous-alcoholic solution, a water-in-oil emulsion, an oil-in-water emulsion, a microemulsion, an aqueous gel, an anhydrous gel, a serum or a dispersion of vesicules, of microcapsules or of microparticles.

9. Use according to any one of the preceding claims, characterized in that the composition contains at least one agent chosen from antibacterial agents, antiparasitic agents, antifungal agents, anti-inflammatory agents, anti-pruriginous agents, anaesthetics, antiviral agents, keratolytic agents, anti-free-radical agents, antiseborrhoeic agents, antidandruff agents, antiacne agents and/or agents which modify the differentiation and/or proliferation and/or pigmentation of the skin.

10. Use according to the preceding claim, characterized in that the agent is lidocaine hydrochloride, an antiparasitic agent or an anti-inflammatory agent which is non-steroidal.

11. Use of at least one CGRP antagonist for preparing a composition containing a dermatologically or pharmaceutically acceptable medium and intended for the topical treatment of skin irritations and/or scabs and/or erythema and/or dysaesthesic sensations and/or sensations of inflammation and/or pruritus of the skin and/or of the mucous membranes.

12. Use according to Claim 11, characterized in that the CGRP antagonist is used in an amount ranging from 0.000001 to 10% by weight relative to the total weight of the composition.

13. Use according to either of Claims 11 and 12, characterized in that the CGRP antagonist is used in an amount ranging from 0.0001 to 5% by weight relative to the total weight of the composition.

14. Use according to any one of Claims 11 to 13, characterized in that the CGRP antagonist is a molecule which reduces the vasodilation induced by capsaicin.

15. Use according to any one of Claims 10 to 14, characterized in that the CGRP antagonist is a molecule which reduces an inhibition of the contraction of the smooth muscle of the deferent canal induced by CGRP.

16. Cosmetic treatment process, characterized in that a composition containing at least one CGRP antagonist in a cosmetically acceptable medium is applied to the skin, to the scalp and/or to the mucous membranes.

17. Cosmetic treatment process for treating sensitive skin-types, characterized in that a composition containing at least one CGRP antagonist in a cosmetically acceptable medium is applied to the skin.

18. Process according to Claim 16 or 17, characterized in that the CGRP antagonist is a molecule which reduces the vasodilation induced by capsaicin.

19. Process according to one of Claims 16 to 18, characterized in that the CGRP antagonist is a molecule which reduces an inhibition of the contraction of the smooth muscle of the deferent canal induced by CGRP.

20. Process according to any one of Claims 16 to 19, characterized in that the CGRP antagonist is chosen from CGRP 8-37 and anti-CGRP antibodies.

21. Process according to any one of Claims 16 to 20, characterized in that the CGRP antagonist is used in an amount ranging from 0.000001 to 10% by weight relative to the total weight of the composition.

22. Process according to any one of Claims 16 to 21, characterized in that the CGRP antagonist is used in an amount ranging from 0.00001 to 5% by weight relative to the total weight of the composition.

23. Topical composition containing a cosmetically, pharmaceutically or dermatologically acceptable medium and at least one product having an irritant side effect, characterized in that it also contains at least one CGRP antagonist such that it reduces the irritant effect of the said product.

24. Composition according to Claim 24, characterized in that the CGRP antagonist is a molecule which reduces the vasodilation induced by capsaicin.

25. Composition according to Claim 23 or 24, characterized in that the CGRP antagonist is a molecule which reduces an inhibition of the contraction of the smooth muscle of the deferent canal induced by CGRP.

26. Composition according to any one of Claims 23 to 25, characterized in that the CGRP antagonist is chosen from organic or inorganic molecules.

27. Composition according to any one of Claims 23 to 26, characterized in that the CGRP antagonist is used in an amount ranging from 0.000001 to 10% by weight relative to the total weight of the composition.

28. Composition according to any one of Claims 23 to 27, characterized in that the CGRP antagonist is used in an amount ranging from 0.0001 to 5% by weight relative to the total weight of the composition.

29. Composition according to any one of Claims 23 to 28, characterized in that the product having an irritant side effect is chosen from α-hydroxy acids, β-bydroxy acids, α-keto acids, β-keto acids, retinoids, anthralins, anthranoids, peroxides, minoxidil, lithium salts, antimetabolites, vitamin D and derivatives thereof, depigmenting agents, solvents, fragrances, preserving agents, surfactants and alcoholic solutions.

30. Composition according to any one of Claims 23 to 29, characterized in that it also contains at least one agent chosen from antibacterial agents, antiparasitic agents, antifungal agents, antiinflammatory agents, anti-pruriginous agents, anaesthetics, antiviral agents, keratolytic agents, anti-free-radical agents, antiseborrhoeic agents, antidandruff agents, antiacne agents and/or agents which modify the differentiation and/or proliferation and/or pigmentation of the skin.

31. Composition according to any one of Claims 23 to 30, characterized in that the composition constitutes a cleansing, protective, treatment, artificial tanning or care composition for the face, the hands, the feet, the major anatomical folds or the body, a make-up product, a make-up-removing milk, an after-sun product, a composition for the bath, a deodorizing composition, a composition for treating wrinkles, acne or hyperseborrhoeic skin or seborrhoeic dermatitis, an aftershave product, a hair-removing cream, a composition to counter insect bites, a pain-relief composition, a composition for hair care or hair treatment, a shampoo composition, a hair setting composition, a styling product composition, a dye composition or a permanent-waving composition, a composition for combating hair loss or a buccodental composition.
